# EUROPÄISCHE PATENTANMELDUNG

(11) **EP 0 841 509 A1**
(43) Veröffentlichungstag der Anmeldung: **13.05.1998**
(21) Anmeldenummer: 97111339.4
(22) Anmeldetag: 04.07.1997
(51) Int. Cl.: F16K 7/04, A62B 9/02

(54) **Flussregulator**

(30) Priorität: 30.08.1996 SE 9603160
(71) Anmelder: Siemens-Elema AB, 171 95 Solna (SE)
(72) Erfinder: Eriksson, Per-Göran, 183 40 Täby (SE); Krahbichler, Erik, 136 62 Haninge (SE); Slettenmark, Bruno, 175 60 Järfälla (SE)

(57) **Zusammenfassung**

Die Erfindung bezieht sich auf einen Flußregulator, vorzugsweise bei einem Respirator/Ventilator, bei dem der Flußregulator einen Schlauch, der von einem Medium, dessen Fluß reguliert werden soll, durchströmt wird, und ein außerhalb des Schlauches angeordnetes Drosselventil umfaßt. Das Drosselventil umfaßt ein erstes Solenoid, ein Druckmittel, das von dem Wellenende des Solenoids beeinflußt wird, sowie einen festen Anschlag zum Beeinflussen des Durchströmungsquerschnitts des Schlauches. Die Welle des Solenoids weist eine derartig wirksame Hublänge auf, daß das Druckmittel in einer ersten Endlage den Durchströmungsquerschnitt des Schlauches unbeeinflußt läßt und in einer zweiten Endlage den Schlauch gegen den Anschlag zusammenpreßt. Um einen Flußregulator dieser Art mit einem Drosselventil zu erhalten, der verhältnismäßig leicht und billig ist und der außerdem einen verhältnismäßig niedrigen Stromverbrauch hat, wird erfindungsgemäß ein zweites Solenoid (4) vorgeschlagen, dessen Welle (6) eine im Vergleich zu der Welle (5) des ersten Solenoids (3) kurze wirksame Hublänge aufweist, und die in der einen Endlage einen derartigen Druck gegen das Druckmittel (8) ausüben kann, daß es den Schlauch (1) weiter zusammenpreßt und dadurch den Fluß ganz unterbindet.

## Beschreibung

Die Erfindung bezieht sich auf einen Flußregulator, vorzugsweise bei einem Respirator/Ventilator, bei dem der Flußregulator einen Schlauch, der von einem Medium, dessen Fluß reguliert werden soll, durchströmt wird, und ein außerhalb des Schlauches angeordnetes Drosselventil umfaßt, das ein erstes Solenoid, ein Druckmittel, das von dem Wellenende des Solenoids beeinflußt wird, sowie einen festen Anschlag zum Beeinflussen des Durchströmungsquerschnitts des Schlauches aufweist, wobei die Welle des Solenoids eine derartig wirksame Hublänge aufweist, daß das Druckmittel in einer ersten Endlage den Durchströmungsquerschnitt des Schlauches unbeeinflußt läßt und in einer zweiten Endlage den Schlauch gegen den Anschlag zusammenpreßt.

Ein Flußregulator dieser Art ist in dem Siemens-Manual "Servo Ventilator 300" gezeigt und ist dafür vorgesehen, den Gasfluß, der den Exspirationsschlauch eines Respirators/Ventilators während einer Ausatmungsphase durchströmt, zu steuern. Während einer Inspirationsphase wird der Schlauch gegen den Anschlag derart zusammengepreßt, daß der Gasfluß ganz unterbunden wird. Da das Solenoid nun nicht nur dafür vorgesehen ist, den Durchströmungsquerschnitt des Schlauches zu steuern, sondern während gut der halben Betriebszeit den Schlauch gegen den Anschlag ganz zusammenzupressen, ist ein Solenoid erforderlich, dessen Welle und damit auch das Druckmittel am Ende der Bewegung eine überproportional große Kraft gegen den Schlauch ausüben kann. Ein Solenoid mit solchen Eigenschaften ist teuer, verhältnismäßig groß und außerdem schwer. Darüber hinaus verbraucht es viel Strom, was nachteilig ist, insbesondere in den Fällen, in denen der Respirator/Ventilator batteriebetrieben ist.

Der Erfindung liegt die Aufgabe zugrunde, einen Flußregulator der eingangs genannten Art mit einem Drosselventil zu schaffen, das verhältnismäßig leicht und billig ist und das außerdem einen verhältnismäßig niedrigen Stromverbrauch hat.

Diese Aufgabe ist erfindungsgemäß dadurch gelöst, daß der Flußregulator ein zweites Solenoid aufweist, dessen Welle eine im Vergleich zu der Welle des ersten Solenoids kurze wirksame Hublänge aufweist und die in der einen Endlage einen derartigen Druck gegen das Druckmittel ausüben kann, daß es den Schlauch weiter zusammenpreßt und dadurch den Fluß ganz unterbindet. Ein solches Solenoid mit einer verhältnismäßig kurzen wirksamen Hublänge von etwa 1 bis 2 mm ist in der Regel ziemlich klein und leicht, kann aber auf dieser kurzen Strecke eine große Kraft ausüben. Mit wirksamer Hublänge ist die Strecke gemeint, in der die Welle des Solenoids aktiv arbeitet. Durch Verwenden eines Solenoids mit diesen Eigenschaften kann ein im Vergleich zu dem bekannten Solenoid schwächeres und dadurch kleineres und leichteres Solenoid verwendet werden, dessen Welle eine solche Kraft aufweist, daß es den Durchströmungsquerschnitt steuern, aber den Schlauch nicht ganz zusammenpressen kann. Somit ist ein Drosselventil gegeben, das leichter ist und einen verhältnismäßig niedrigen Stromverbrauch hat.

Solenoide der genannten Art sind in dem Handbuch "Solenoids Design Manual" der Fa. SHINDENGEN ELECTRIC MFG. CO., LTD. gezeigt und beschrieben.

In einer vorteilhaften Ausführungsform der Erfindung wird vorgeschlagen, daß das zweite Solenoid derart bemessen ist, daß es eine derartige Kraft gegen den Schlauch erzeugen kann, daß es selbst den Schlauch gegen den Anschlag ganz zusammenpressen kann. Hierdurch ist erreicht, daß der Strom für das erste Solenoid ausgeschaltet werden kann, wenn der Schlauch gegen den Anschlag ganz zusammengepreßt ist. Somit wird ein extrem niedriger Stromverbrauch erreicht.

Die Wellen der Solenoide können nach der Erfindung parallel angeordnet sein und über ein Verbindungsmittel miteinander verbunden werden.

Im Hinblick auf eine vorteilhafte Weiterbildung der Erfindung wird vorgeschlagen, daß das Verbindungsmittel als Druckmittel dient. Das Verbindungsmittel kann vorzugsweise auch mit dem Druckmittel verbunden sein.

In einer weiteren vorteilhaften Ausgestaltung der Erfindung wird vorgeschlagen, daß die Solenoiden in Serie angeordnet sind, wobei die Welle des zweiten Solenoids derart angeordnet ist, daß diese die Welle des ersten Solenoids beeinflussen kann. Hierdurch ist ein verhältnismäßig kompaktes und dadurch platzsparendes Drosselventil gegeben.

In einer Weiterentwicklung des Drosselventils nach der Erfindung wird vorgeschlagen, daß die Solenoide in Serie angeordnet sind und eine gemeinsame Welle aufweisen. Somit wird die Anzahl der Teile des Drosselventils auf ein Minimum gebracht.

Die Erfindung ist nachfolgend anhand mehrerer in den Zeichnungen dargestellten Ausführungsbeispielen näher erläutert. Es zeigen:

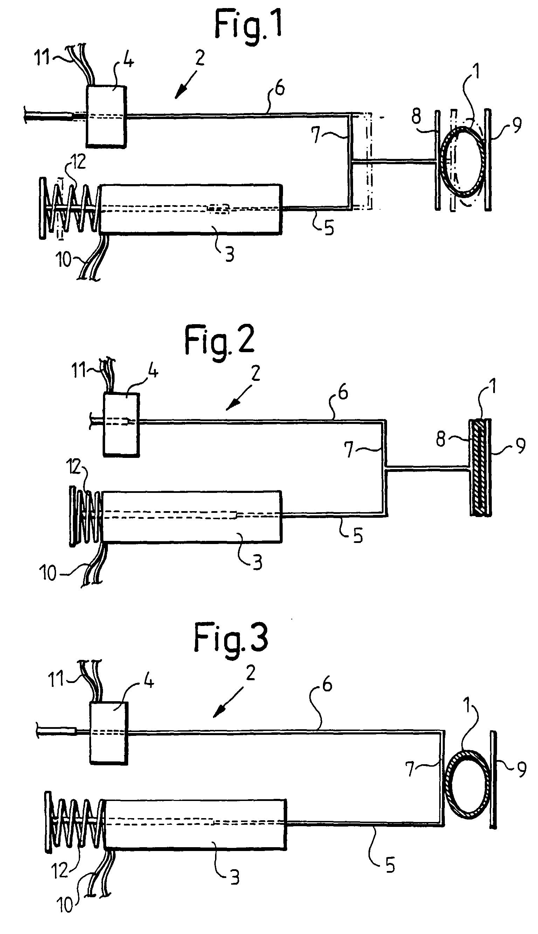

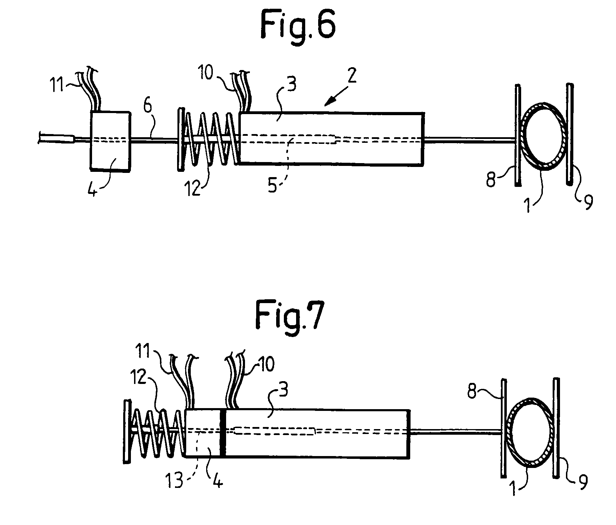

FIG. 1 bis 7 schematisch gezeichnete Flußregulatoren nach der Erfindung in verschiedenen Ausführungsformen und in verschiedenen Lagen.

Der Flußregulator nach FIG. 1, der vorzugsweise in Verbindung mit einem Respirator/Ventilator verwendet wird, umfaßt einen Exspirationsschlauch 1, der von einem Medium, dessen Fluß reguliert werden soll, durchströmt wird, und ein außerhalb des Schlauches 1 angeordnetes Drosselventil 2. Das Gas kommt von einem hier nicht gezeigten Patienten. Das Drosselventil 2 weist ein erstes und ein zweites Solenoid 3, 4 auf, die derart angeordnet sind, daß deren Wellen 5, 6 parallel verlaufen und über ein Verbindungsmittel 7 miteinander verbunden sind. Das Verbindungsmittel 7 ist seinerseits mit einem Druckmittel 8 verbunden. Das Drosselventil 2 umfaßt auch einen festen Anschlag 9, wobei der Exspirationsschlauch 1 zwischen dem Anschlag 9 und dem Druckmittel 8 angeordnet ist. Die Wellen 5, 6 der Solenoide 3, 4 sind teils aus einem magnetischen und teils aus einem nichtmagnetischen Material hergestellt. In sämtlichen Figuren sind die Strecken der Wellen 5, 6, die stärker gezeichnet sind, aus einem magnetischen Material. Das erste Solenoid 3 ist größer als das zweite Solenoid 4 und weist auch eine wesentlich größere wirksame Hublänge auf.

In der FIG. 1 ist gezeigt, daß das gemeinsame Druckmittel 8 der Solenoide 3, 4 in einer ersten Endlage den Durchströmungsquerschnitt des Schlauches 1 unbeeinflußt läßt. Indem dem Solenoid 3 über Leitungen 10 mehr oder weniger Strom zugeführt wird, wird der Teil der Welle 5, der aus einem magnetischen Material hergestellt ist, derart beeinflußt, daß er so weit in den Solenoidkörper hineingezogen wird, daß das Verbindungsmittel 7 und das Druckmittel 8 gegen den Schlauch 1 drückt. Auf diese Weise kann der Durchströmungsquerschnitt des Schlauches 1 variiert werden, so daß ein gewünschter Gasfluß erhalten wird. In der Figur ist mit Hilfe der strichpunktierten Konturen ein Beispiel einer solchen Lage gezeigt. Gleichzeitig damit, daß die Welle 5 verschoben wird, wird auch die Welle 6 des zweiten Solenoids 4 passiv die gleiche Strecke verschoben, so daß sich der Teil der Welle 6, der aus einem magnetischem Material hergestellt ist, dem Solenoidkörper nähert, was auch mit Hilfe von strichpunktierten Konturen der Welle 6 gezeigt ist. In diesem Ausführungsbeispiel weist das erste Solenoid 3 eine derartig wirksame Hublänge auf, daß es mit Hilfe des Druckmittels 8 den Schlauch 1 bis auf einige Millimeter ganz zusammenpressen kann. In dieser Lage, in der die Strecke der Welle 6, die aus einem magnetischen Material hergestellt ist, nun den Solenoidkörper erreicht hat, wird dem Solenoid 4 über Leitungen 11 Strom zugeführt. Die Welle 6 des Solenoids 4, die eine wirksame Hublänge aufweist, die der Strecke entspricht, die erforderlich ist, um den Schlauch 1 ganz zusammenzudrücken, wird nun derart aktiviert, eine solche Kraft gegen den Schlauch 1 auszuüben, daß dieser wie es in der FIG 2 dargestellt ist, ganz zusammengepreßt wird. Da das Solenoid 4 bemessen ist, eine solche Kraft gegen den Schlauch 1 erzeugen zu können, daß es alleine diesen gegen den Anschlag 9 ganz zusammenpressen kann, kann der Strom zum ersten Solenoid 3 ausgeschaltet werden, wobei die Welle 5 des Solenoids 3 durch das Verbindungsmittel 7 diese letzte Strecke lediglich passiv verschoben wird. Das Solenoid 4 hält nun während einer Einatmungsphase den Schlauch 1 in einer ganz zusammengedrückten Lage. Bei einer nachfolgenden Ausatmungsphase wird die Stromzufuhr zum Solenoid 4 ausgeschaltet, wobei dessen Welle 6 kraftlos wird. Eine Druckfeder 12 sieht zu, daß die Wellen 5, 6 und damit auch das Druckmittel 8 in die ursprünglich beschriebene Lage verschoben wird.

Im Rahmen der Erfindung kann das erste Solenoid 3 derart bemessen sein, daß die wirksame Hublänge der Welle 5 zwar reicht, um den Schlauch 1 ganz zusammenpressen zu können; es hat aber nicht genügend Kraft für eine solche Zusammenpressung. Das zweite Solenoid 4 kann derart bemessen sein, daß es den Schlauch 1 nicht alleine gegen den Anschlag 9 ganz zusammenpressen kann, aber daß die Solenoide 3 und 4 während einer Einatmungsphase gemeinsam den Schlauch 1 in einer solchen Lage halten können.

In der FIG 3 ist gezeigt, daß das erwähnte Verbindungsmittel 7, das in Verbindung mit den FIG. 1 und 2 beschrieben wird, selbst als Druckmittel dienen kann.

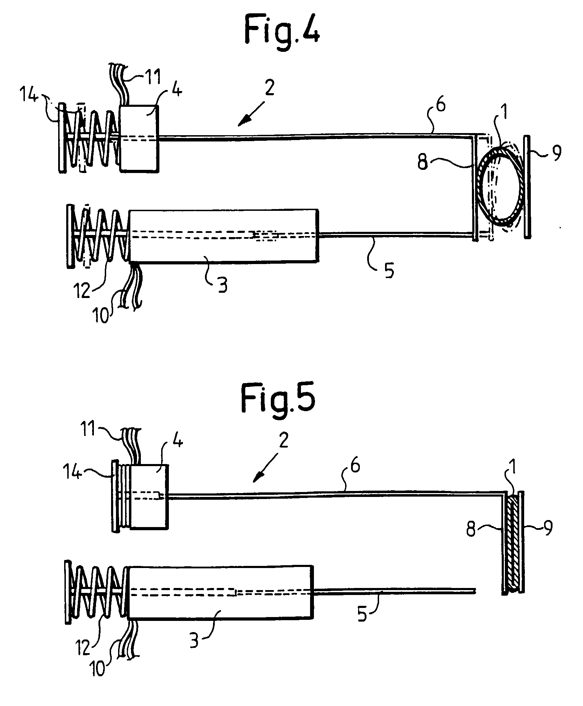

In den FIG. 4 und 5 soll dargestellt werden, daß lediglich die Welle 6 des Solenoids 4 mit dem Druckmittel 8 fest verbunden sein kann. Der Flußregulator arbeitet auf die gleiche Weise wie es in Verbindung mit den FIG. 1 und 2 beschrieben wird mit den Unterschied, daß nach durchgeführter Arbeit die Stromzufuhr zum Solenoid 3 ausgeschaltet wird. Dies bedeutet, daß, wenn die Welle 6 des Solenoids 4 bzw. das Druckmittel 8 auf beschriebene Weise aktiviert wird, den Schlauch zusammenzupressen, die Welle 5 durch die Druckfeder 12 in ihre ursprüngliche Lage zurückverschoben wird. Eine solche Lage ist in der FIG. 5 gezeigt. Wenn die Stromzufuhr zum Solenoid 4 danach ausgeschaltet wird, sieht eine Druckfeder 14 zu, daß die Welle 6 dieses Solenoids 4 und damit auch das Druckmittel 8, wie es in der FIG. 4 gezeigt ist, in ihre ursprünglichen Lagen zurückverschoben werden.

In der FIG. 6 ist gezeigt, daß die Solenoide 3 und 4 statt parallel in Serie angeordnet sind, wobei die Welle 6 des zweiten Solenoids 4 so angeordnet ist, daß sie die Welle 5 des ersten Solenoids 3 beeinflussen kann. Im übrigen arbeitet der Flußregulator auf die gleiche Weise wie es in Verbindung mit den FIG. 1 und 2 beschrieben worden ist. Durch diesen Aufbau ist das Druckmittel 8 direkt mit der Stirnseite der Welle 5 lösbar verbunden.

In der FIG. 7 ist dargestellt, daß die in Serie angeordneten Solenoide 3, 4 auch eine gemeinsame Welle 13 haben können.

Das Wesentliche bei der Erfindung ist es, daß durch Verwendung von zwei kleinen Solenoiden mit verschieden wirksamen Hublängen ein insgesamt kleiner, leichter und billiger Flußregulator gegeben ist, der verhältnismäßig wenig Strom verbraucht und der insbesondere dann extrem wenig Strom verbraucht, wenn das kleinere der Solenoide eine derartig große Kraft gegen den Schlauch ausüben kann, daß es diesen allein gegen den Anschlag zusammenpressen kann derart, daß die Stromzufuhr zu dem größeren der beiden Solenoiden ausgeschaltet werden kann.

### Bezugszeichenliste

- 1: Exspirationsschlauch
- 2: Drosselventil
- 3: ein erstes Solenoid
- 4: ein zweites Solenoid
- 5, 6, 13: Welle
- 7: Verbindungsmittel
- 8: Druckmittel
- 9: Anschlag
- 10, 11: Leitung
- 12, 14: Druckfeder

## Patentansprüche

1. Flußregulator, vorzugsweise bei einem Respirator/Ventilator, bei dem der Flußregulator einen Schlauch, der von einem Medium, dessen Fluß reguliert werden soll, durchströmt wird, und ein außerhalb des Schlauches angeordnetes Drosselventil umfaßt, das ein erstes Solenoid, ein Druckmittel, das von dem Wellenende des Solenoids beeinflußt wird, sowie einen festen Anschlag zum Beeinflussen des Durchströmungsquerschnitts des Schlauches aufweist, wobei die Welle des Solenoids eine derartig wirksame Hublänge aufweist, daß das Druckmittel in einer ersten Endlage den Durchströmungsquerschnitt des Schlauches unbeeinflußt läßt und in einer zweiten Endlage den Schlauch gegen den Anschlag zusammenpreßt, **dadurch gekennzeichnet**, daß der Flußregulator ein zweites Solenoid (4) aufweist, dessen Welle (6) eine im Vergleich zu der Welle (5) des ersten Solenoids (3) kurze wirksame Hublänge aufweist und die in der einen Endlage einen derartigen Druck gegen das Druckmittel (8) ausüben kann, daß es den Schlauch (1) weiter zusammenpreßt und dadurch den Fluß ganz unterbindet.

2. Flußregulator nach Anspruch 1, **dadurch gekennzeichnet**, daß die Wellen (5, 6) der Solenoide (3, 4) parallel angeordnet und über ein Verbindungsmittel (7) miteinander verbunden sind.

3. Flußregulator nach Anspruch 2, **dadurch gekennzeichnet**, daß das Verbindungsmittel (7) als Druckmittel (8) dient.

4. Flußregulator nach Anspruch 2, **dadurch gekennzeichnet**, daß das Verbindungsmittel (7) mit dem Druckmittel (8) verbunden ist.

5. Flußregulator nach Anspruch 1, **dadurch gekennzeichnet**, daß die Solenoide (3, 4) in Serie angeordnet sind, wobei die Welle (6) des zweiten Solenoids (4) derart angeordnet ist, daß diese die Welle (5) des ersten Solenoids (3) beeinflussen kann.

6. Flußregulator nach Anspruch 1, **dadurch gekennzeichnet**, daß die Solinoide (3, 4) in Serie angeordnet sind und eine gemeinsame Welle (13) aufweisen.

7. Flußregulator nach Anspruch 1, **dadurch gekennzeichnet**, daß die Wellen (5, 6) der Solenoide (3, 4) parallel miteinander angeordnet sind, wobei lediglich die Welle (6) des zweiten Solenoids (4) mit dem Druckmittel fest verbunden ist.

8. Flußregulator nach einem der Ansprüche 1 bis 7, **dadurch gekennzeichnet**, daß das zweite Solenoid (4) derart bemessen ist, daß es eine derartige Kraft gegen den Schlauch (1) erzeugen kann, daß es selbst den Schlauch (1) gegen den Anschlag (9) ganz zusammenpressen kann.
